# EUROPEAN PATENT APPLICATION

(11) **EP 0 709 304 A1**
(43) Date of publication of application: **01.05.1996**
(21) Application number: 95307500.9
(22) Date of filing: 20.10.1995
(51) Int. Cl.: B65D 81/20, A61J 1/00

(54) **Package for a veterinary implant**

(30) Priority: 26.10.1994 US 329562
(71) Applicant: AMERICAN HOME PRODUCTS CORPORATION, Madison, New Jersey 07940-0874 (US)
(72) Inventor: Chaudhry,Sharda, Fremont, California 94539 (US); Ash, Robin Taffy, Cupertino, California 95014 (US); Lee, Jung-Chung, San Jose, California 95148 (US); Miksztal, Andrew R., Menlo Park, California 94025 (US); Lee, Min Long, Saratoga, California 95070 (US)
(74) Representative: Wileman, David Francis, Dr.

(57) **Abstract**

A package for a trenbolone-containing veterinary implant comprises a trenbolone-containing veterinary implant, a sealed barrier container surrounding the implant, and an inert gas filling the sealed container.

## Description

### Field of the Invention:

This invention relates to a package for a veterinary implant.

### Description of the Field:

Veterinary implants containing steroids are well-known for the enhancement of weight gain in beef cattle. They are typically a number (e.g. three to twelve) of (usually) cylindrical pellets, prepared by compression of the steroid active ingredient(s) with binders, lubricants, colorants, etc. Implants may be administered by subcutaneous or intramuscular implantation; usually by subcutaneous implantation in the ear, using an implanter specifically designed for the purpose. For convenience in implantation, several implants are typically held in an implant magazine designed to fit the implanter; and typical magazine designs are clips, trays or belts, each holding a number of implants in a single row, and cylinders, holding a number of implants around the periphery. An implanter and associated cylindrical implant magazine are described in U.S. Patents Nos. 4,400,170 and 4,474,572 (McNaughton et al.); and other implanters, implant magazines, and implants are well-known in the veterinary pharmaceutical field. The disclosures of these and other documents referred to throughout this application are incorporated herein by reference.

Trenbolone 17β-hydroxyestra-4,9,11-trien-3-one, sometimes also referred to as trienbolone or trienolone, is an anabolic steroid described in U.S. Patent No. 3,248,194 (Nomine et al.). Veterinary implants containing trenbolone or trenbolone derivatives in combination with estradiol or estradiol derivatives are described in U.S. Patent No. 3,939,265 (Grandadam); and similar implant formulations containing trenbolone or trenbolone derivatives in combination with zeranol are described in U.S. Patent No. 4,192,870 (Grandadam et al.).

In the United States, veterinary implants containing trenbolone acetate, 17β-acetoxyestra-4,9,11-trien-3-one, are marketed as Finaplix^{®}, and veterinary implants containing trenbolone acetate and 17β-estradiol are marketed as Revalor^{®}, both by Hoechst-Roussel Agri-Vet Company. These implants are labeled for storage at refrigeration temperatures (2-8°C), and have an apparent shelf life of 1½ - 2 years under those conditions. However, to store implant products under refrigeration is an inconvenient and expensive process for both pharmacies and, especially, farmers; and it would be desirable to develop an implant product that is stable for an extended period at room temperature.

### SUMMARY OF THE INVENTION

We have discovered that trenbolone and trenbolone esters, such as trenbolone acetate, and veterinary implants containing them as an active ingredient are susceptible to both oxidative and photocatalyzed degradation; and that the stability of these implants can be greatly enhanced by protecting the implants through packaging under inert gas in a sealed barrier container.

One aspect of the present invention is a package for a trenbolone-containing veterinary implant, where the package comprises a trenbolone-containing veterinary implant, a sealed barrier container surrounding the implant, and an inert gas filling the sealed container.

Another aspect of this invention is a method of enhancing the stability of a trenbolone-containing veterinary implant, the method comprising the steps of placing the implant within a barrier container, filling the container with an inert gas, and sealing the container.

The package of the invention provides several advantages:
most importantly, the package enhances the stability of the implant, extending its shelf life and eliminating the need for refrigerated storage;
additionally, the package acts to protect the implant from physical damage during handling and storage; and
the package is easily and economically manufactured, since technology for such packaging is readily commercially available and relatively inexpensive.

### BRIEF DESCRIPTION OF THE DRAWINGS

Figure 1 shows the degradation of trenbolone-containing implants packaged under air or nitrogen and stored at 40°C.

Figure 2 shows the degradation of trenbolone-containing implants packaged under air or nitrogen and stored at 25°C.

### DETAILED DESCRIPTION OF THE INVENTION

### Definitions

A "trenbolone ester" is an ester of trenbolone with an aliphatic, alicyclic, or aromatic carboxylic acid of 1 to 10 carbon atoms. A preferred trenbolone ester is trenbolone acetate.

A "trenbolone-containing veterinary implant" is a solid dosage form for implantation containing trenbolone or trenbolone ester as an active ingredient. The implant may contain other active ingredients, such as estrogenic steroids, especially 17β-estradiol ("estradiol") or its esters such as 17β-estradiol-3-benzoate ("estradiol benzoate"), or zeranol; and excipients such as binders, lubricants, colorants, and the like. A preferred implant contains trenbolone acetate as an active ingredient, either alone or in combination with estradiol or estradiol benzoate.

"Implant" includes individual pellets, unit dosage forms (which are conventionally referred to as "implants") containing several pellets, and implant magazines filled or partially filled with implants, as the context requires.

An "implant magazine" or "magazine" is a device, typically in the form of a clip, tray, belt, or cylinder, capable of retaining a number of implants, and from which the implants may be implanted into an animal.

"Barrier containers" are containers constructed of materials such that, when filled with inert gas and sealed, they effectively prevent contact of the contents of the container with atmospheric oxygen or light, and thereby prevent or minimize oxidative and photocatalyzed degradation of the contents.

"Inert gas" refers to gases of low chemical reactivity suitable for package filling, such as nitrogen and the rare gases (especially argon). A preferred inert gas is nitrogen. "Filling" a barrier container with inert gas and sealing it implies that the sealed container be essentially free of oxygen, *i.e.,* that oxygen be so sufficiently removed and excluded from the container that oxidative degradation of a contained trenbolone-containing veterinary implant be minimized. Because the oxidative degradation of the implant is to a certain extent dependent on the ratio of residual oxygen in the barrier container to the amount of trenbolone in the container, and this is dependent on the volume of the container and the number of contained implants, it is not practicable to specify a particular oxygen concentration limit as being uniformly satisfactory to minimize degradation. However, one of ordinary skill in the art will have no difficulty, having regard to that skill and this disclosure, in determining a suitable maximum oxygen concentration for any container and contents. For example, for implants packaged in the foil pouches of Examples 1 and 2 of this invention, a suitable residual oxygen concentration in the sealed container is less than 1%, preferably less than 0.5%, and more preferably less than 0.25%.

### Description

Barrier containers are well-known in the packaging art; and are widely used, for example, in the food-service industry for the storage of prepackaged foods susceptible to oxidative or photocatalyzed degradation *(e.g.* pouches of ground coffee, gable-top cartons of fruit juice/concentrate, etc.). Such containers are typically prepared from polymeric co-extrusions in which one or more of the polymeric layers is a "barrier polymer", customarily defined as a polymer having an oxygen permeability of less than 10 cm³ · mil/(100 in. · d · atm) [38.9 cm³ · µm/(m · d · kPa)] at 23°C. Barrier polymers and their uses are described, for example, in the article entitled "Barrier Polymers" in the *The Wiley Encyclopedia of Packaging Technology,* M. Baker, Ed., John Wiley and Sons, New York, 1986. Such containers are also typically prepared from foil laminates (polymeric laminates in which one or more of the layers is a metal foil, especially an aluminum foil, or a metalized polymeric layer); and may also contain other non-polymeric layers such as paperboard.

Barrier containers may be made in many forms, but those forms particularly applicable to this invention are preformed or form-fill-and-seal pouches (where the container is flexible), lidded trays (where the tray is rigid or semi-rigid, and may be shaped particularly to retain the implants/implant magazines), and gable-top cartons; all of which are readily applicable to inert gas filling and sealing.

The trenbolone-containing implant is placed in the barrier container, which is then filled with the inert gas (the term "flushed" is frequently used in the packaging art to describe the replacement of one gas, especially the ambient gas such as air with another, such as an inert gas; and "filled" as used here included "flushed") and sealed. This is typically performed by placing the container in a chamber which is evacuated to remove all oxygen and is then refilled with the chosen inert gas at an appropriate pressure. While in the chamber, the container is then sealed by any suitable means, typically by heat sealing (heating either by direct conduction, applicable to all materials, or by inductive heating, applicable if a metal foil or foil laminate is present in the area of the container to be sealed).

A particular convenient barrier container for the package of this invention is a pouch formed from a foil laminate (such as the Maraflex foil laminate described in the Examples below); as these containers may be commercially obtained preformed (already sealed on three sides) and may be conveniently loaded with the trenbolone-containing implants and filled with inert gas and sealed by semi-automatic or automatic machinery, such as by the use of the packaging machine described in the Examples below.

The pressure of the inert gas filling the sealed barrier container is not critical to the practice of this invention. However, especially when the barrier container is flexible or semi-rigid, such as a pouch or lidded tray, it is convenient that the pressure be approximately atmospheric pressure, typically between 0.8 and 1.2 atmospheres, preferably between 0.9 and 1.1 atmospheres.

Suitable materials for such containers, and filling and sealing techniques are well-known in the packaging art. They are described, for example, in *The Wiley Encyclopedia of Packaging Technology,* referred to above, and equipment is widely commercially available, so that one of ordinary skill in the art will have no difficulty, having regard to that skill and this disclosure, in determining suitable materials and techniques for the practice of this invention.

The stability of a trenbolone-containing veterinary implant packaged in the package of this invention may be determined by analysis of the implants for the content of trenbolone or trenbolone ester, as appropriate, by conventional analytical techniques.

By the use of the package of this invention, the stability of trenbolone-containing veterinary implant is enhanced so that the implant may be stored without refrigeration at "room temperature" *(e.g.* at 20-25°C) or even at temperatures up to 40°C for periods of two years or more while still retaining acceptable potency, that is, the packaged implant has a shelf life exceeding two years.

### EXAMPLES

### Example 1. Single Magazine Pouch

A cylindrical magazine of the type described in U.S. Patent No. 4,400,170, made from low density polyethylene, was loaded with ten implants. Each implant consisted of eight cylindrical pellets; each pellet being 3.1 mm in diameter and 3.8 mm in length and containing 25 mg trenbolone acetate and 3.5 mg estradiol benzoate, together with polyethylene glycol 8000, povidone, magnesium stearate, and red colorant.

A pouch 133 mm long and 114 mm wide was formed of a foil laminate [Maraflex M-4637, American Can Company: 12 µm polyethylene terephthalate outer layer, 17 µm white low density polyethylene, 18 µm aluminum foil, and 38 µm duPont Surlyn 1652 (a sodium-neutralized ethylene/methacrylic acid ionomer) inner sealing layer] and factory sealed to a depth of 9.5 mm on three sides. The magazine was placed in the pouch, and the pouch containing the magazine was placed into the chamber of a Turbovac Model SB-320H vacuum packaging machine with the open end across the sealing bar. The packaging machine, previously attached to the nitrogen gas cylinder, was operated using an automated sealing cycle that evacuated the sealing chamber, filled it with nitrogen at one atmosphere, and heat sealed the pouch. On completion of the sealing operation, the sealed pouch was allowed to cool, and the seal inspected for leaks and other defects. The package atmosphere was analyzed for oxygen content, and the oxygen concentration found to be 0.2%.

### Comparative Example 1.

Trenbolone-containing veterinary implants, loaded in a cylindrical magazine, were packaged and heat-sealed in a pouch identical to that of Example 1, except that the pouch was filled with air, rather than the nitrogen of Example 1.

### Experiment 1. Stability Determination

Trenbolone-containing veterinary implants packaged as in Example 1 (nitrogen-filled) and as in Comparative Example 1 (air-filled) were studied for stability under various storage conditions. In each case, a number of pouches were prepared and stored at controlled temperatures. At appropriate times, pouches were removed from controlled-temperature storage and opened, and the implants analyzed for trenbolone acetate content. In the analysis determination, the implant was dissolved in tetrahydrofuran and the solution diluted to a suitable concentration with acetonitrile, than made up to contain 50% v/v water. The resulting solution was analyzed by HPLC, using a Metachem Inersil ODS-25µ column and was eluted with a step gradient of water and acetonitrile (75% water initially to 15% water finally), detecting the trenbolone and degradation products by UV absorption at 231 nm.

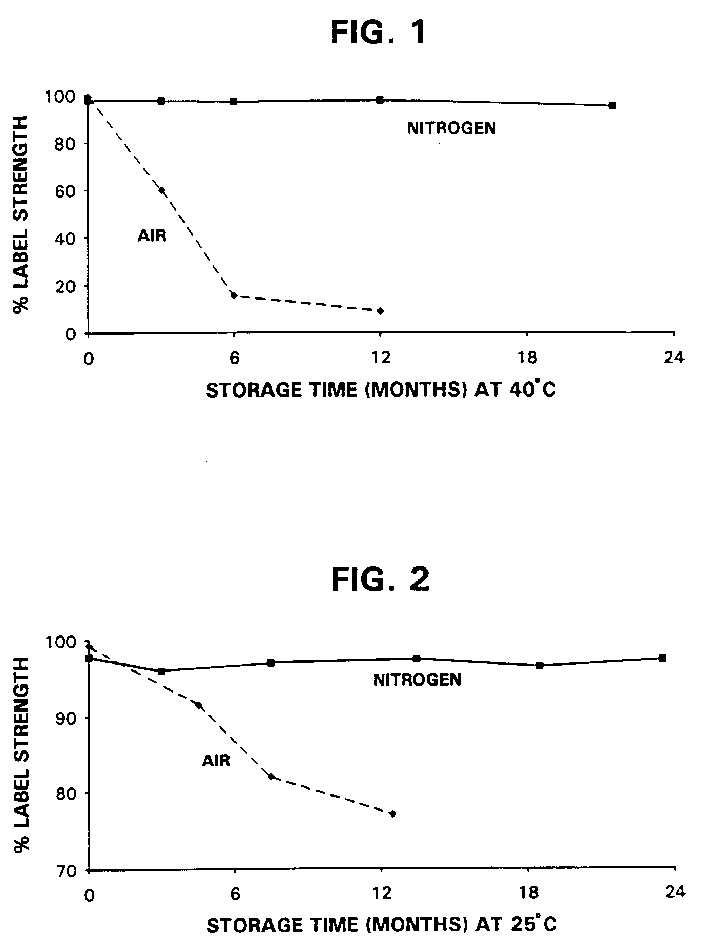

Figures 1 and 2 show the results of stability studies carried out at 40°C and 25°C, respectively. In each Figure, the horizontal axis represents the storage time for the packaged implant in months at the chosen temperature, and the vertical axis represents the percentage of label strength of trenbolone acetate in the implant, *i.e.* the trenbolone acetate content of the implant as actually measured given as a percentage of the indicated (label) trenbolone acetate content, 25 mg per pellet. Measurements for implants packaged as in Example 1 (nitrogen-filled) are shown by the solid lines, with sampling and analysis points indicated by squares, and the stability of these implants was studied for up to two years. Measurements for implants packaged as in Comparative Example 1 (air-filled) are shown by the dashed lines, with sampling and analysis points indicated by diamonds, and the stability of these implants was studied for up to one year.

Figure 1 shows that at 40°C the trenbolone acetate in implants of Example 1 maintained over 95% label strength for two years, while the trenbolone acetate in implants of Comparative Example 1 quickly deteriorated to less than 20% label strength within six months.

Figure 2 shows that at 25°C the trenbolone acetate implants of Example 1 maintained over 95% label strength for two years, while the trenbolone acetate implants of Comparative Example 1 deteriorated to less than 80% label strength within one year.

These results clearly show the superior storage stability of trenbolone-containing veterinary implants packaged as in the present invention.

### Example 2. Ten Magazine Pouch

Ten cylindrical magazines were filled with trenbolone acetate/estradiol benzoate implants as described in Example 1. A pouch 229 mm long and 178 mm wide was made of Maraflex M-4637, as described in Example 1; and the magazines placed inside. The pouch was evacuated, filled with nitrogen, and sealed using a Turbovac Model SB-320H vacuum packaging machine as in Example 1. On completion of the sealing operation, the sealed pouch was allowed to cool, the seal inspected for leaks and other defects.

Trenbolone-containing veterinary implants packaged as described in Example 2 exhibited sufficient storage stability for a shelf-life exceeding two years at room temperature.

### Example 3. Other Packages

Trenbolone-containing veterinary implants may also be packaged in other inert gas filled sealed barrier containers.

A gabletop carton, sized to hold magazines as described in Example 1, is formed from a six-ply laminate (polyethylene outer layer, paperboard, ionomer, aluminum foil, ionomer, low-density polyethylene inner sealing layer). The carton is loaded with trenbolone-containing veterinary implants in magazines (as described in Example 1), evacuated, nitrogen filled, and sealed.

A barrier tray, sized to hold magazines as described in Example 1, is thermoformed from a five-ply polymer laminate (opaque polypropylene, adhesive, ethylene/vinyl alcohol copolymer as barrier layer, adhesive, polypropylene). The tray is loaded with trenbolone-containing veterinary implants in magazines, lidded with Maraflex M-4637 foil laminate, evacuated, nitrogen filled and sealed.

Other trenbolone-containing veterinary implants and implant magazines, and other barrier containers are inert gases suitable for packaging of trenbolone-containing veterinary implants and implants under inert gas, may also be employed.

While this invention has been described in specific embodiments and examples, a person of ordinary skill in the art will understand that various modifications may be made and equivalents may be substituted without departing from the invention, and all such modifications and equivalents are intended to be within the scope of the claims.

## Claims

1. A package for a trenbolone-containing veterinary implant, comprising:
(a) a trenbolone-containing veterinary implant,
(b) a sealed barrier container surrounding the implant, and
(c) an inert gas filling the sealed container.

2. A package according to Claim 1 where the implant comprises trenbolone acetate.

3. A package according to Claim 2 where the implant is selected from trenbolone acetate, trenbolone acetate/estradiol, and trenbolone acetate/estradiol benzoate.

4. A package according to any one of Claims 1 to 3 where the container is a pouch.

5. A package according to Claim 4 where the pouch is constructed of a foil laminate comprising at least one metal foil layer and at least one polymeric layer.

6. A package according to Claim 5 where the metal foil is aluminium foil.

7. A package according to any one of Claims 1 to 6 where the package is heat sealed.

8. A package according to any one of Claims 1 to 7 where the pressure of the inert gas filling the container is approximately one atmosphere.

9. A package according to any one of Claims 1 to 8 where the inert gas is nitrogen.

10. A package according to any one of Claims 1 to 9 where the implant is contained in a magazine.

11. A process for preparing a trenbolone-containing veterinary implant, the method comprising the steps of:
(a) placing the implant in a barrier container,
(b) filling the container with an inert gas, and
(c) sealing the container e.g. by heat treatment.
